# EUROPEAN PATENT APPLICATION

(11) **EP 1 600 175 A1**
(43) Date of publication of application: **30.11.2005**
(21) Application number: 04012818.3
(22) Date of filing: 28.05.2004
(51) Int. Cl.: A61L 9/12, B60H 3/00, A61L 9/03

(54) **Air filter and fragrance vapourizer**

(71) Applicant: Hsieh, Hai-Fang, Taoyuan County (TW); Chen, Yueh-A, Taoyuan County (TW)
(72) Inventor: Hsieh, Hai-Fang, Taoyuan County (TW); Chen, Yueh-A, Taoyuan County (TW)
(74) Representative: Zeitler, Giselher

(57) **Abstract**

The present invention discloses an improved air filter structure, comprising a container, a housing covering the container, a motor, a fan, and a stirring rod disposed at the bottom of the fan. A plant liquid or an aromatic liquid is placed in the container, and the gas outside the housing circulate continuously in and out of the container by the fan, such that the molecules of the liquid in the container can be released quickly to fill the space of a room with fragrance. A plurality of sensors is disposed at appropriate positions of the housing, and connected to a control switch. When someone is approaching the sensing range, the sensor will turn on the control switch and start the motor, so that the fan will circulate the airflow, and the stirring rod will stir the liquid to accelerate the dispersion of the liquid molecules. The air entering the container will be in touch with the surface of the liquid, so that the dust originally mixed in the air will be attached on the surface of the liquid and sunk to the bottom of the container by gravitation, and thus achieving the effect of filtering the impurities in the air.

## Description

### BACKGROUND OF THE INVENTION

### Field of the Invention

The present invention relates to air filters, more particularly to an improved air filter structure for filtering the impurities in the air of a room and filling the room with fragrance, which contains a plant liquid or an aromatic liquid in a container, and brings the molecules of the liquid to the room by the air circulation produced by a fan, and lets the air in the container touch the liquid surface, and thus can achieve the effect of filtering impurities. The air filter further has a sensor for automatically detecting a person who is approaching the range of the sensor to control and start the operation of a motor.

### Description of the Related Art

A bad smell in a room with its windows and doors closed or inside a car usually makes us feel uncomfortable. Some airtight spaces are not equipped with ventilation facilities, so that the air inside is almost isolated completely from the outside without any ventilation and the smell is unable to disperse. Therefore, many people put an air freshener in the airtight space, and the aromatic molecules of the air freshener are spread into the air to achieve the effects of suppressing the smell and reducing the bad feeling of the smell.

However, a conventional air freshener generally disperses the molecules into the air by evaporation. The effect created by such evaporation method is slow and limited, and thus some manufacturers add wood extracts to air fresheners to improve the evaporation effect. These wood extracts have adverse effect on seriously jeopardizing our health. Further, although some air fresheners directly spray aromatic chemicals into the air by an accumulator (an air compressed can) or a fan, the spray cannot be circulated in the air continuously, and thus can have a temporary effect only.

### Summary of the Invention

The primary objective of the present invention is to provide an improved air filter structure that makes use of a container, a housing covering the container, a motor, a fan, and a stirring rod disposed at the bottom of the fan to give an air freshening effect. The air filter contains a plant liquid or an aromatic liquid in the container and the air outside the housing continues to circulate in and out of the container by the fan, so that the molecules of the liquid contained in the container are discharged and the room is filled with fragrance more quickly.

Further, a plurality of sensors is installed at appropriate positions of the housing, and the sensor is connected to a control switch, so that when someone is approaching the range of the sensor, the sensor turns on the control switch to start the motor and at the same time the stirring rod stirs the liquid to accelerate the dispersion of the molecules of the liquid. The air in the container touches the liquid surface, and the dust in the air adheres to the liquid surface and then sinks to the bottom of the container by gravitation, and thus achieving the effect of filtering impurities in the air.

### BRIEF DESCRIPTION OF THE DRAWINGS

Other features and advantages of the present invention will become apparent in the following detailed description of the preferred embodiments with reference to the accompanying drawings, in which:
FIG. 1 is a view of the external look of the air filter structure of the present invention.
FIG. 2 is a view of the disassembled parts of the air filter structure of the present invention.
FIG. 3 is a cross-sectional view of the air filter structure of the present invention.
FIG. 4 is a view of the engagement of the fan and stirring rod according to the present invention.
FIG. 5 is a view of the external look of the air filter structure according to another preferred embodiment of the present invention.
FIG. 6 is a view of the air filter structure according to the present invention when it is in use.

### DETAILED DESCRIPTION OF THE PREFERRED EMBODIMENTS

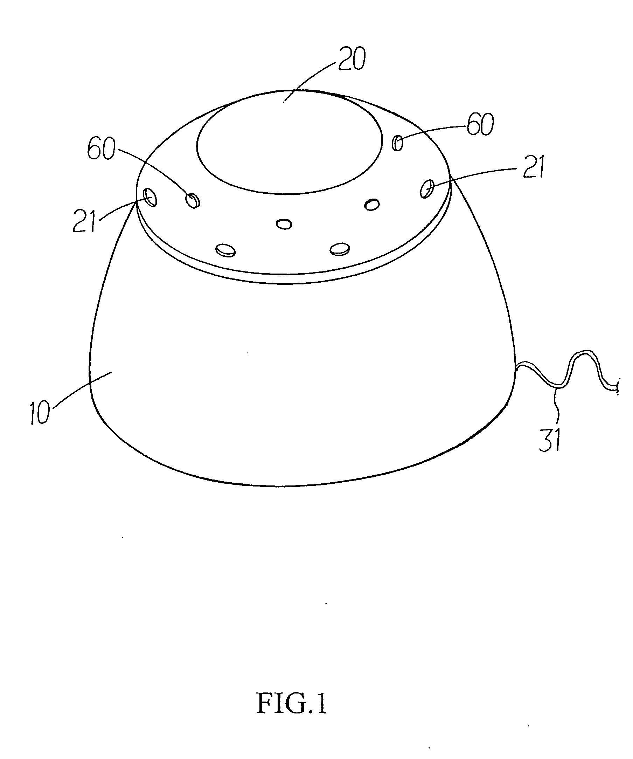

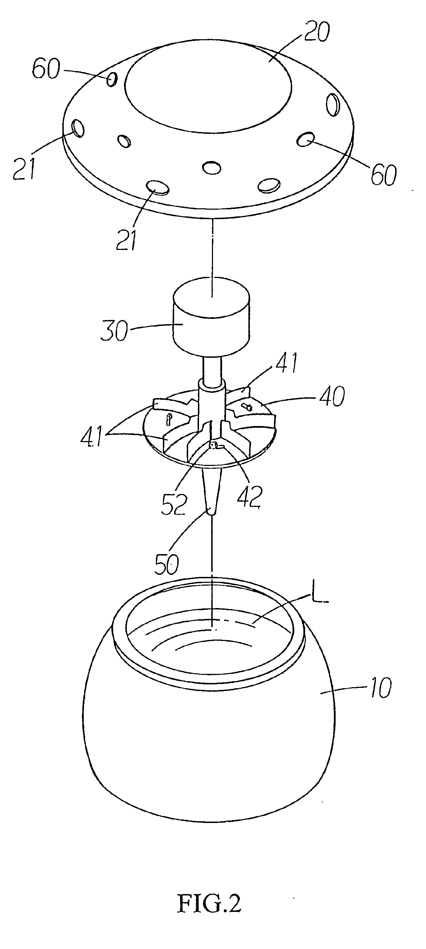

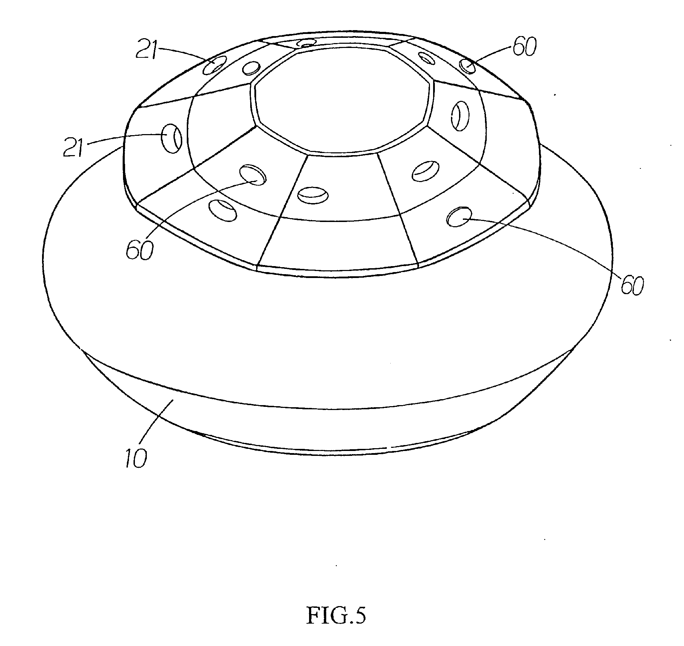

The improved air filter structure in accordance with the present invention as shown in FIGS. 1 and 2 comprises: a container 10 for containing a liquid L; a housing 20 covering the top of the container 10 and having an air passage 21 for letting air enter and exit the container; a motor 30 disposed in the housing with its core axle facing the liquid L contained in the container 10, a fan 40 disposed on the core axle; a stirring rod 50 disposed under the fan 40 and dipped into the liquid L of the container 10. The air filter according to a preferred embodiment of the invention shown in the figure is coupled by a control switch (not shown in the figure) and a power cable 31 to electrically connect the whole air filter to a power supply. Of course, the whole air filter can have a space (not shown in the figure) in the housing 10 for accommodating a battery, so that the motor is coupled to a power cable of the battery and a control switch to control the power supply of the battery and drive the operation of the air filter. The air filter could be in any shape, such as having the shape of "Eight Diagrams in Chinese mythology" for the housing 20 as shown in FIG. 5.

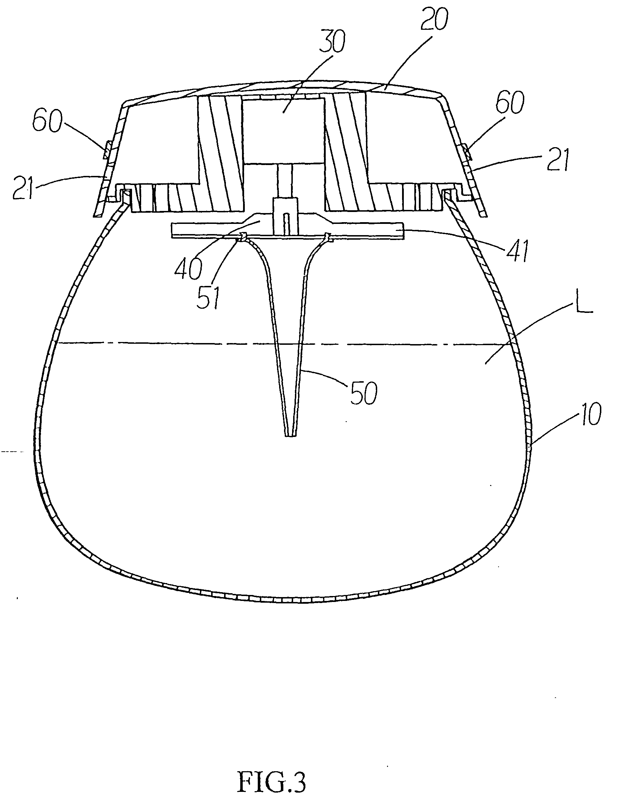

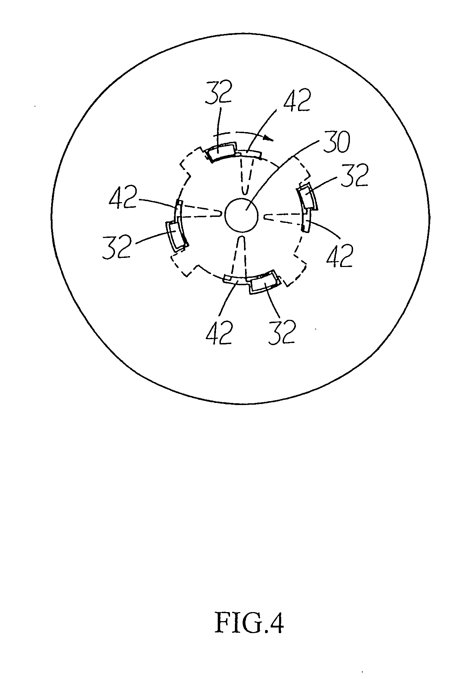

Further, refer to FIGS. 2 and 4. In the figures, the fan 40 comprises a disc structure having a vane 41 at its top, and a stirring rod 50 at its top has a flange 51 coupled to the bottom of the fan 40. A latch groove 42 and a latch member 52 are disposed respectively on the disc member of the fan 40 and the flange 51 of the stirring rod 50 for the engagement with each other, so that the stirring rod 50 extends into the latch groove 42 of the fan 40 by the latch member 52, and the latch member 52 and the latch groove 42 are coupled securely by the rotation to constitute the coupling of the stirring rod 50 and the fan 40. Further, the container 10 is preferably made of a transparent material as shown in FIG. 3 for letting the user know about the storage level of the liquid L in the container 10 and the rotation condition of the fan 40 and the stirring rod 50.

While the fan 40 is driving the air circulation, the stirring rod 50 also rotates to stir the liquid L as the fan 40 rotates in order to improve the activity of the molecules of the liquid L. More particularly, the air entering into the container 10 from the outside of the housing 20 will touch the surface of the liquid L, so that the dust originally mixed in the air attaches on the surface of the liquid L, and the dust sinks to the bottom of the container 10 by gravitation, and thus achieving the effect of filtering impurities from the air.

Further, a plurality of sensors 60 is disposed at appropriated positions of the housing 20, and the sensor could be a photoelectric cell which emits an infrared ray to detect whether an object falls into the range of the sensor. The sensor is connected to a control switch, so that when someone is approaching the sensing range, the sensor turns on the control switch to start the motor. Please refer to FIG. 3 at the same time. In the actual operation of the whole air filter, a liquid L such as a plant liquid or an aromatic liquid is put into the container 10, and the motor 30 drives the fan 40 to rotate and produce an air circulation. The air outside the housing 20 is brought into the container by the air circulation and then discharged from the container to the outside of the housing 20 according to the air circulation. Therefore, the air outside the housing 20 keeps circulating in and out of the container 10 and discharges the molecules of the liquid L contained in the container 10 quickly to fill the room with fragrance.

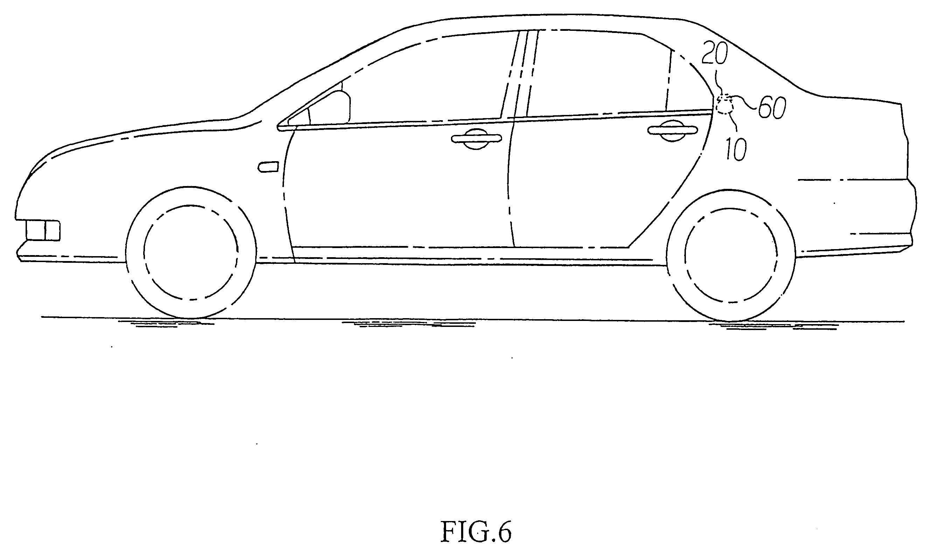

Of course, the air filter could be installed in a car as shown in FIG. 6. When a person enters into the car, the sensor 60 senses the person's approaching the car to turn on the control switch and start the motor, and further drives the operation of the air filter. On the other hand, when a person is leaving the car, the sensor 60 no longer detects the existence of human body in the car, and then the control switch controls and stops the motor, and further stops the operation of the air filter. Such air filter can automatically detect whether or not a person is approaching in order to control the motor operation.

In summation of the description above, the "Improved air filter structure" of the invention aims at the improvement of the conventional air filter by adding a plurality of sensors at appropriate positions of the housing, so that the sensors detect an approaching human body to start the operation of the air filter, and filter the impurities in the air of the room, and fill the room with fragrance. The present invention herein enhances the performance than the conventional structure and further complies with the patent application requirements and is submitted to the Patent and Trademark Office for review and granting of the commensurate patent rights.

While the present invention has been described by the most practical and preferred embodiments, it is understood that this invention is not limited to the disclosed embodiments but is intended to cover various arrangements included within the spirit and scope of the broadest interpretations and equivalent arrangements.

## Claims

1. An improved air filter structure, comprising: a container for containing a liquid; a housing for covering the top of said container; an air passage for letting air flow in and out of said container; a motor being installed in said housing with its core axle facing the liquid in said container, and said core axle further comprising a fan, a stirring rod disposed at the bottom of said fan and dipped into the liquid in said container, and a control switch for controlling the rotation of said motor; wherein a liquid selected from the collection of a plant liquid and an aromatic liquid being disposed in said container, and said fan producing an air circulation to bring the air from the outside of said housing into said container, and discharge the air in the container out of said housing by said air circulation, thereby the air outside said housing being circulated in and out of said container and liquid molecules contained in said container being discharged quickly to fill a room with fragrance, and while said fan producing an air circulation, said stirring rod stirring the liquid in said container as said fan rotates, and thus improving the activity of liquid molecules to accelerate the dispersion of liquid molecules and the contact of air entering said container from the outside of said housing with the liquid surface and attach the dust originally mixed in the air onto the liquid surface, and the dust sinking to the bottom of said container due to gravitation, and thus achieving the effect of filtering the impurities in the air, **characterized in that**:
a plurality of sensors, being disposed at appropriate positions of said housing and coupled to a control switch; thereby when a person approaching the sensing range of said sensor, said sensor turning on said control switch to start said motor, and thus achieving the effect of filtering air.

2. The improved air filter structure of claim 1, wherein said fan is a disc structure having a vane at its top, and said stirring rod comprises a flange disposed at the top of said stirring rod and coupled to the bottom of said fan, and the disc structure of said fan and the flange of said stirring rod respectively have a latch groove and a latch member to extend into and fix said stirring rod into the latch groove of said fan and engage said latch member with said latch groove by a rotation to couple said stirring rod and said fan.

3. The improved air filter structure of claim 1, wherein said container is made of a transparent material.

4. The improved air filter structure of claim 1, wherein said motor is coupled to a power cable of said control switch to electrically connect an air filter.

5. The improved air filter structure of claim 1, wherein said housing has a space for accommodating a battery, and said motor is coupled to a power cable of said control switch to drive the operation of said air filter by a battery.

6. The improved air filter structure of claim 1, wherein said sensor is a photoelectric cell which emits an infrared ray to sense and determine an object falling into a sensing range of said sensor.
